# EUROPEAN PATENT SPECIFICATION

(11) **EP 2 146 993 B1**
(45) Date of publication and mention of the grant of the patent: **05.08.2015**
(21) Application number: 08736038.4
(22) Date of filing: 10.04.2008
(51) Int. Cl.: C07D 471/14

(54) **A METHOD FOR THE PREPARATION OF MIRTAZAPINE**
METHODE ZUR HERSTELLUNG VON MIRTAZAPIN
PROCÉDÉ DE PRÉPARATION DE MIRTAZAPINE

(30) Priority: 11.04.2007 EP 07105985
(43) Date of publication of application: 27.01.2010
(73) Proprietor: Merck Sharp & Dohme B.V., 2031 BN Haarlem (NL)
(72) Inventor: KEMPERMAN, Gerardus Johannes, NL-5340 BH Oss (NL)
(74) Representative: Hussain, Deeba
(86) International application number: PCT/EP2008/054316
(87) International publication number: WO 2008/125578

(56) References cited:
- WO-A-2005/005410
- WO-A-2007/144409
- US-A- 4 062 848

## Description

The present invention relates to a method for the preparation of a tetracyclic benzazepine, in particular of mirtazapine, in substantial enantiomeric excess of the R or S form. The invention further relates to a novel intermediate and its use for the preparation of mirtazapine having a substantial enantiomeric excess of the R or S form. The invention further relates to a method for the preparation of an intermediate tetracyclic compound having substantial enantiomeric excess of the R or S form.

Mirtazapine (1,2,3,4,10,14b-hexahydro-2-methyl-pyrazino[2,1-a]pyrido[2,3c][2]benzazepine) is a tetracyclic compound having the formula IV:

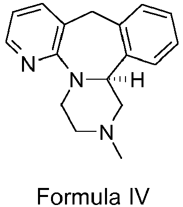

The compound is chiral and the racemic mixture finds widespread use as a medicine for the treatment of depression. Other medical uses for mirtazapine have also been reported e.g., WO 99/25356 and WO 01/58453 disclose its use in the treatment of sleep disorders and apnea. Investigations into the biological effects of the enantiomers of mirtazapine (e. g. O'Connor and Leonard, Neuro-pharmacology, 1986, vol. 25, pp.267-270 ; Kooyman et al., 1994, vol. 33, pp. 501-507 ; De Boer et. al., Neuro-pharmacology, 1988, vol. 27, pp. 399-408 ; Gower et al., 1988, vol. 291, pp 185-201) mention the compound in its pure enantiomeric forms. There is a need to make the enantiomers, in particular the S-mirtazapine available in large quantities. The present invention provides for improvement of an efficient production of large quantities of enantiomerically pure mirtazapine and related benzazepines.

A variety of methods are known in the art for the preparation of mirtazapine. US 4062848 describes variations within a four stage synthetic scheme by which the synthesis of mirtazapine can be accomplished starting from a 2-substituted nicotinitrile. Further modifications to various stages of this route have subsequently been described in WO 00/62782, WO 01/23345 and US 6,376, 668.

According to the method described in US4062848 mirtazapine can be obtained as a result of ring closure of a compound of formula (A), 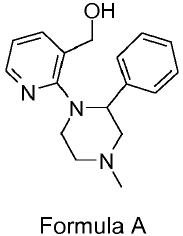 Examples of such agents include acids such as sulfuric acid, concentrated hydrochloric acid, picric acid, trifluoroacetic acid, phosphoric acid, polyphosphoric acid (PPA), phosphorus oxychloride, phosphorus pentoxide and Lewis Acids such as aluminium chloride, ferric chloride, zinc chloride, tin chloride, titanium chloride, boron trifluoride, antimony pentachloride and zirconium tetrachloride. In US4062848 preparation of mirtazapine is exemplified by ring closure using concentrated sulfuric acid. In WO00/62782 it is indicated that concentrated sulfuric acid is most preferred.

The optical resolution of racemic mirtazapine has also been addressed in US4062848. By the method disclosed in US4062848, enantiomerically pure mirtazapine is obtained by forming diastereomeric salts by reaction of racemic mirtazapine with enantiomerically pure dibenzoyltartaric acid in ethanol, filtering off the thus formed diastereomeric salt, followed by regeneration of the free base by treatment with aqueous ammonia. In this method resolution of the enantiomers occurs at the end of the synthetic pathway after having manufactured a racemic mixture of mirtazapine. It follows therefore that the overall yield of each enantiomerically pure compound obtained is relatively low and can never be more than 50%. Another drawback of such a late stage enantiomer separation is that significant amounts of waste are generated. It would be beneficial to have a more economic and environmentally benign method in which enantiomerically pure mirtazapine could be prepared with an overall improved yield.

In US4062848 a remark is made that pure enantiomers of mirtazapine might also be obtained synthetically by using optically active precursors for the last ring closure step. However, it was found that the method described in US4062848 and WO00/62782 with concentrated sulfuric acid does not sufficiently retain optical purity. Apparently, those reaction conditions allow excessive racemisation.

In document WO 2005/005410 it is described that for the synthesis of enantiomerically pure mirtazapine by ring closure of an enantiomerically pure compound of above formula (A), stereochemical integrity in the starting material can be preserved by making a specific selection out of the above mentioned ring closing reagents. The method comprises forming mirtazapine with enantiomeric excess comprising ring closure of a compound according to formula (A) having enantiomeric excess by treatment with a suitable acid in the absence of a solvent or a suitable combination of an acid and an organic solvent.

The inventors have found that the reaction described in WO 2005/005410 can proceed without substantial racemization by selection of the appropriate conditions, but that under such conditions a significant amount of side products is formed that complicates purification of the enantiopure mirtazapine and consequently deteriorates the overall yield of the process. Reversely, the formation of side products can be prevented only at the cost of loss of optical purity. It is therefore an object of the invention to provide a method for the preparation of mirtazapine, in particular of mirtazapine in enantiomerically pure form or with an enantiomeric excess of the R or S form that does not have the above drawbacks.

This object has been achieved in the method according to the invention for the preparation of mirtazapine (formula IV) having greater than 50% enantiomeric excess of the R or S form, said method comprising the steps of
a: providing a carboxylic acid compound according to Formula I having greater than 50% enantiomeric excess of the R or S form,
b: converting the carboxylic acid group of compound I into a ketone group, producing a ketone compound of Formula II,
c: optionally reducing ketone compound II with a metal hydride or a borohydride to form the intermediate hydroxy compound of Formula III and
d: forming the mirtazapine of Formula IV by reduction of the ketone compound II or of the hydroxy compound III using as the reducing agent a mixture of a metal hydride and a metal halogenide or a mixture of a borohydride and a strong acid.

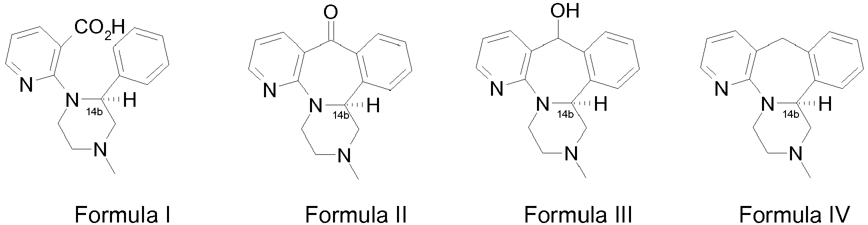

This reaction was found to proceed without any substantial racemization and without formation of any significant amount of side products. The reaction product can be used for preparing pharmaceutical preparations without the necessity of further purification or of optical resolution. The method of the invention enables the production of mirtazapine and formulations thereof having an enantiomeric excess of the R or S form of at least 80% and comprising side products in an amount below 10 wt% more preferably less than 5 weight percent and most preferably less than 2 weight percent (wt% expressed as the total weight of the side products divided by the total weight of the obtained reaction product x 100%). Preferably the enantiomeric excess of the desired enantiomer is at least 90%, more preferably more than 95%.

The term mirtazapine is used here in the generic meaning that is commonly used to refer to the chemical compound as a base but also to the salts and solvates thereof. The term mirtazapine as used in the description can refer to the S form, the R form or the racemic form, as will be clear from the context in which the term is used. The term mirtazapine supplemented with the prefixes (R) or (S) specifically refers to the enantiomers of the compound. The structure formulas incorporated in this description depicting one particular form are intended to cover the S form, the R form and the racemic form unless specifically indicated otherwise.

The compound with Formula III comprises, apart from the main chiral centre at carbon atom 14b, an additional chiral centre at the carbon atom to which the hydroxyl group is attached. Consequently of this compound four stereoisomers can exist comprising two diastereoisomers, viz. (R,R), (R,S), (S,R) or (S,S). However, in this description the indication R or S form only refers to the absolute configuration at the chiral centre that is present at carbon atom 14b in the middle ring adjacent to the nitrogen.

The term "enantiomeric excess" refers to the difference between the amounts of each of the enantiomers present in a mixture, relative to the total amount of the compound in the mixture expressed as percentage (x 100%). In separate embodiments the term "substantial enantiomeric excess" refers to an enantiomeric excess of more than 50%, preferably more than 70%, more preferably more than 80%, even more preferably more than 90% and most preferably more than 95%. The term "enantiopure" or "substantially enantiopure" implies in separate embodiments an enantiomeric excess of at least 80%, more preferably at least 90%, even more preferably more than 95%.

The term "side product" refers to products in the obtained reaction product other than mirtazapine (in S or R form) or its unreacted intermediate. The presence of side products does not influence the enantiomeric excess of an enantiomer of a compound of the invention, as the enantiomeric excess is calculated solely with the amounts of enantiomers of the compounds of the invention. The amount of side products however is relevant for the utilization of the obtained mirtazapine as biologically active compounds. As the removal of substantial amounts of unwanted side products is costly and time consuming, the amount of side products should be as low as possible.

### Detailed description of the method of the invention

### Step a

The method of the invention starts with a carboxylic acid with Formula I in substantial enantiomeric excess or preferably in substantially enantiopure form. Such compound can for example be obtained by diastereomeric crystallisation of the racemic mixture as described in US4062848.

### Step b

In this step the carboxylic acid group of compound I is converted into a ketone group, producing a ketone compound of Formula II. Preferably the carboxylic acid group is first activated with an activator and subsequently reacted with a Lewis acid. The activator is preferably a chlorinating agent, preferably thionyl chloride, oxalyl chloride or phosphorus oxychloride. This activation leads to an intermediate acid chloride compound. This compound is then reacted with a Lewis acid. The Lewis acid preferably is a metal halogenide, preferably aluminum chloride, tin chloride, iron chloride or zinc chloride. It was found that this reaction step proceeds without substantial racemisation and that the ketone intermediate compound of Formula II could be obtained maintaining the enantiomeric excess of the carboxylic acid compound, in particular even in a substantially enantiopure form.

### Steps c and d

In one embodiment, the ketone with Formula II can be first reduced with a weak reducing agent in a separated step (c) to the alcohol with Formula III, which alcohol is then, in a subsequent step, reduced to mirtazapine with Formula IV. In an alternative preferred embodiment, the ketone with Formula II can be reduced in a one step reaction with a strong reducing agent directly to mirtazapine of Formula IV. It is believed that the reduction of the ketone with Formula II to mirtazapine with Formula IV in a one step reaction proceeds via the intermediate alcohol of Formula III. In this case the alcohol is not obtained as a separate intermediate product, but is believed to be present in the reaction mixture of the reduction.

In optional step c, the ketone compound of Formula II is reduced to an intermediate hydroxyl compound with Formula III that can be isolated. This reduction is a mild reduction. The reducing agent suitable for this mild reduction step (c) is a metal hydride or a borohydride. Prefered metal hydrides are alkali metal hydride, preferably lithium aluminum hydride, diisobutylaluminum hydride, lithium tri-tert-butoxyaluminumhydride, sodium bis(2-methoxyethoxy)aluminumhydride, etc. Prefered borohydrides are alkaliborohydrides, preferably sodium borohydride, lithium borohydride, lithium tri-sec-butylborohydride, lithium triethylborohydride, sodium triacetoxyborohydride etc.

As described above, the hydroxyl compound formed in step (c) is expected to be a mixture of diastereomers, as this compound with Formula III is a diastereomeric compound having two chiral centres. However, the absolute configuration at the second chiral center (that of the hydroxyl group) is not relevant for the preparation of the end product.

In final step (d) the ketone compound II of step (b) or hydroxy compound III of step (c) are reduced using a strong reducing agent. Preferably, the strong reduction agent comprises a mixture of a metal hydride reduction agent as described above in step (c) and a metal halogenide, preferably a mixture of an aluminum hydride and an aluminum halogenide, preferably in a molar ratio between 2.5 and 3.5 such that the reducing agent alane (aluminium trihydride) is generated in situ to act effectively as the reducing agent. Preferred metal hydride are alkali metal hydrides, preferably lithium aluminum hydride or diisobutyl aluminum hydride. An alternative strong reduction agent is a mixture of a borohydride and a strong acid, preferably having the acid in molar excess, preferably a mixture of sodium borohydride and methanesulfonic acid or sulfuric acid.

During any of the process steps of the method of the invention dicalite can be added to the reaction mixture to prevent lump formation. The reaction is carried out in a solvent. The solvent type is chosen in view of the chosen reactants and reaction conditions. In case of metalhydride reducing agents, the solvent is aprotic to prevent reaction with the solvent, for example tetrahydrofuran. In case of borohydride a protic solvent can be chosen, for example sulfuric acid/water. Generally, the reaction temperatures can be varied between -30°C and the reflux temperature of the solvents used. The reaction temperature is preferably higher than 40°C to get an acceptable reaction rate. On the other hand the reaction temperature is chosen not too high to prevent side product formation, typically below about 100 °C.

Eventually the product mirtazapine can be crystallized as a free base or as a salt with a biological acceptable counter ion as is known in the art. The obtained product can then be used in the preparation of a pharmaceutical preparation without further optical resolution steps or purification steps.

US 4, 062, 848 describes a ketone compound as a precursor to prepare mirtazapine and makes a broad general statement that optically active compounds can be made from optically active (enantiopure) precursors. The document does not describe how to get substantially enantiopure precursors and does not disclose that, in a specific reduction step as specified in the present invention, such substantially enantiopure precursors can be converted to mirtazapine whilst maintaining the high enantiomeric excess and at a low impurity level. All examples of preparing enantiopure mirtazapine involve resolution of the final product. Further, inventors have found that reduction methods described in this prior art document result in considerable racemization, so the broad statement is merely an unsubstantiated desideratum. Therefore, the disclosed method in this prior art document does not lead to specific mirtazapine precursor hydroxyl compound III or mirtazapine in substantially enantiopure form. The general statement therefore does not provide a specific pointer to enantiopure intermediate compound of formula II. The hydroxyl compound according to formula III in racemic or other form has not been described. Therefore, the invention also relates to the ketone compound according to formula II ((14bS or R)-1,3,4,14b-tetrahydro-2-methyl-pyrazino[2,1-a]pyrido[2,3-c][2]benzazepin-10(2H)-one) in a mixture having at least 50% enantiomeric excess of the R or S form or in enantiopure R or S form and to the hydroxyl compound according to formula III (1,2,3,4,10,14b-hexahydro-2-methylpyrazino[2,1-a]pyrido[2,3-c][2]benzazepin-10-ol) in a mixture having at least 50% enantiomeric excess of the R or S form or in enantiopure form. In particular, to such compounds having enantiomeric excess of the R or S form of at least 80%, preferably at least 95% and comprising side products in an amount below 10 wt%, preferably below 2 wt%. The invention also relates to the use of said compounds for the preparation of mirtazapine (formula IV) having greater than 50% enantiomeric excess of the R or S form.

In view of avoiding undesired side effects in use, for example as a anti-depressant, it is important that the mirtazapine is as pure as possible, that is having a high enantiopurity and low side product content. The mirtazapine obtained by the method according to the invention meets this requirement and therefore is most suitable for use in a pharmaceutical formulation. The invention therefore also relates to a pharmaceutical formulation comprising mirtazapine having an enantiomeric excess of the R or S form of at least 80%, preferably at least 95% and comprising side products thereof in an amount below 10 wt%, preferably below 2 wt% in the form of the base or in the form of a pharmaceutical acceptable salt thereof.

The intermediate alcohol compound III obtained in step (c), 1,2,3,4,10,14b-hexahydro-2-methylpyrazino[2,1-a]pyrido[2,3-c][2]benzazepin-10-ol, is a novel compound which can advantageously be used for the preparation of mirtazapine. The compound can optionally be prepared according to different methods than described above. The invention also relates to a method for the preparation of mirtazapine by reduction of compound III with strong reducing agents as described above, in particular for mirtazapine having a substantial enantiomeric excess of the R or S form. The invention is also directed to the use of a compound having Formula III, preferably in substantially enantiopure R or S form, for the preparation of mirtazapine, preferably having enantiomeric excess of the R or S form.

The method according to the invention is specifically developed for the preparation of mirtazapine, but has been found to be equally applicable for the preparation of other tetra cyclic compounds. Therefore, there is also disclosed a method for the preparation of a tetracyclic compound according to formula VI, having substantial enantiomeric excess of the S or R form, wherein, instead of the carboxylic compound of formula I, a carboxylic compound according to formula V having substantial enantiomeric excess of the S or R form, is used in steps a-d of the method of the invention described above, forming a compound of Formula VI wherein Y and X and Z represent ring structures comprising five to eight ring member atoms and wherein ring structure Y preferably represent an aromatic ring optionally comprising heteroatoms, preferably pyridine or benzyl.

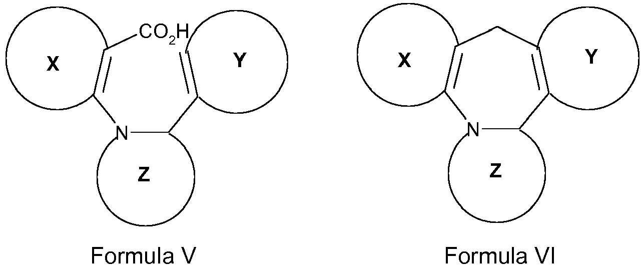

Ring structure X preferably also represents an aromatic ring structure optionally comprising heteroatoms, preferably pyridine or benzyl. Ring structure Z preferably is a partially unsaturated or saturated ring comprising five to eight ring member atoms, preferably comprising a second nitrogen atom in the ring. Ring structures X, Y and/or Z may comprise one or more substitutions each independently selected from alkyl, aryl, alkyl-aryl or alkoxy optionally comprising one or more unsaturated bonds or one or more hetero atoms or halogen, hydroxy or sulfur containing groups.

All the reagents and other conditions described above for steps a to d of the method of the invention for the preparation of mirtazapine in substantial enantiomeric excess of the R or S form, apply accordingly to the method of the invention for the preparation of a tetracyclic compound according to formula VI, having substantial enantiomeric excess of the S or R form.

The invention is illustrated by the following Examples.

### Example 1

### Preparation of (S)-2-(4-methyl-2-phenyl-1-piperazinyl)-3-pyridinecarboxylic acid (compound I)

The enantiopure carboxylic acid, 2-(4-methyl-2-phenyl-1-piperazinyl)-3-pyridinecarboxylic acid (compound I), can be prepared starting from (S)-1-methyl-3-phenylpiperazine and 2-chloro-3-cyanopyridine as shown in the scheme below. In order to obtain significant quantities of (S)-1-methyl-3-phenylpiperazine for use in the synthesis of (S)-mirtazapine, a classical resolution using (S)-(+)-Anicyphos was exploited (see Figure 1). From a series of resolving agents, this compound appeared the most effective. First the salt of (S)-1-methyl-3-phenylpiperazine with (S)-(+)-Anicyphos was crystallized from water. The free base of (*S*)-1-methyl-3-phenylpiperazine was liberated by treatment with sodium hydroxide and extraction with ethyl acetate. In this way (*S*)-1-methyl-3-phenylpiperazine was obtained in an overall yield between 35-40% and with an ee >98%. Alternatively (*S*)-1-methyl-3-phenylpiperazine can be obtained via an enzymatic resolution of the racemic mixture, according to WO2007/144409. Lastly, a stereoconvergent synthesis of (*S*)-1-methyl-3-phenylpiperazine starting from (*S*)-phenylglycine can be accomplished via processes that have been described for the racemate in WO 2003/024918, 2003 and JP2007/284358.

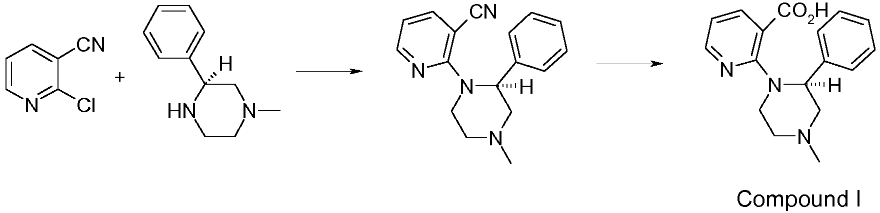

### (S)-1-methyl-3-phenylpiperazine.(+)Anicyphos salt

100 g of (*R,S*)-1-methyl-3-phenylpiperazine (1) (567 mmole) and 154.5 g of (+)-Anicyphos (571 mmole) were dissolved in 250 ml of water by heating the mixture to reflux. After cooling down to room temperature a seed crystal was added. After two hours, the white crystals formed were collected by filtration and dried in a vacuum oven at 40 °C for 21 hours. This provided 121 g (48 %) with an ee of 85.5%. The crystals were dissolved in water (119 ml) at reflux temperature. After cooling down the crystallization started. After one hour the crystals were collected by filtration and dried in a vacuum oven at 40 °C. The yield of the crystals of (S)-1-methyl-3-phenylpiperazine.Anicyphos salt was 105.8 g (42 %, ee 99.0%).

The ee was determined by HPLC analysis: Chiralcel OD 250*4.6mmID (Daicel), 5 % iso-propylalcohol in hexane, flow rate 1.0 ml.min⁻¹, UV-detector, column temperature 40 °C, retention times 5.6 min, 6.3 min.

### (S)-1-methyl-3-phenylpiperazine

(S)-1-methyl-3-phenylpiperazine.(+)Anicyphos salt (100 g) was suspended in dichloromethane (378 ml). To this stirred suspension 25% ammonium hydroxide (63.2 ml) diluted with water (315 ml) was added. The layers were separated. The dichloromethane layer was washed three times with saturated aqueous solution of sodium chloride. The dichloromethane layer was dried with magnesium sulphate, and evaporated to dryness. This yielded 30.3 g (75%, ee 99.0%) of (S)-1-methyl-3-phenylpiperazine as colourless oil that crystallized upon standing.
¹H-NMR data (CDCl₃): δ (ppm): 1.90 (s, 1 H, NH), 2.0 (t,1H, CHₐₓ), 2.16 (dt, 1 H, CHₐₓ), 2,32 (3H, s, CH₃), 2.85 (m, 1H, CH_{eq}), 2.89 (m, 1H, CH_{eq}), 3.12 (dt, 1H, CHₐₓ), 3.15 (m, 1 H, CH_{eq}), 3.88 (dd, 1 H, CH_{benzyl}), 7.23-7.41 (m, 5H, Ar-H).
The ee was determined by HPLC analysis: Chiralcel OD 250*4.6mmID, 10 µm (Daicel), 5 % iso-propylalcohol in hexane, flow rate 1.0 ml.min⁻¹, column temperature 40 °C, UV-detector (210 nm), retention times 5.8 min and 6.7 min.

### 2-[(2S)-4-methyl-2-phenyl-1-piperazinyl]-3-pyridinecarbonitrile

A mixture of (*S*)-1-methyl-3-phenylpiperazine (21.7 g, 123 mmole), 2-chloronicotinitrile (21.7 g, 157 mmole), KF (21.7 g, 373 mmole), and DMF (65 ml) was heated to reflux temperature for 23.5 hours. The DMF was evaporated and to the crude product ethyl acetate (45 ml) and water (64 ml) of 60 °C were added. The mixture was refluxed for 10 minutes. The aqueous layer was separated from the organic layer and was extracted with ethyl acetate (45 ml). The collected ethyl acetate layers were evaporated to dryness. This furnished 2-[(2S)-4-methyl-2-phenyl-1-piperazinyl]-3-pyridinecarbonitrile (53.5 g, >100%, ee 97.6%) as a brown oil that was directly used in the subsequent step.
¹H-NMR data (CDCl₃): δ (ppm): 2.38 (s, 3H, CH₃), 2.57 (m, 1H, CH), 2.78 (m, 2H), 2.95 (dd, 1 H, CH), 3.6 (m, 1 H, CH), 5.43 (t, 1 H, CH_{benzyl}), 6.79 (dd, 1H, Ar-H), 7.18 (m, 1 H, Ar-H), 7.26 (m, 2H, Ar-H), 7.37 (m, 1 H, Ar-H), 7.78 (dd, 1 H, Ar-H), 8.26 (dd, 1 H, Ar-H).
The ee was determined by HPLC analysis: Chiralcel OD-H 250*4.6mmID, 10 µm (Daicel), 3 % iso-propylalcohol in hexane+0.1 % diethylamine, flow rate 1.0 ml.min⁻¹, column temperature 40 °C, UV-detector (295 nm), retention times 6.6 min and 7.7 min.

### 2-[(2S)-4-methyl-2-phenyl-1-piperazinyl]-3-pyridinecarboxylic acid

2-[(2S)-4-methyl-2-phenyl-1-piperazinyl]-3-pyridinecarbonitrile (53.5 g) was dissolved in methanol (86.8 ml) and the solution was heated to 50 °C. A 33% sodium hydroxide solution (127.4 ml) was added and the reaction mixture was refluxed for three days. The mixture was diluted with water (22 ml) and the methanol was evaporated. Thus formed oil was separated from the salty aqueous phase and dissolved in water (96 ml) by increasing the temperature to 80 °C. The pH was adjusted 5.5 ± 0.5 by adding sulphuric acid and the solution was stirred for 15 minutes. Water was removed by coevaporation with toluene (320 ml). The solution was filtered to remove the salts. The filtrate was evaporated to dryness and the crude product was co-evaporated twice with a mixture of methanol (87 ml) and water (2 ml). The residue was dissolved in acetone (109 ml) at 50 °C after which the solution was cooled to ambient temperature at which it was stirred overnight. The mixture was stirred for another hour at -10 °C after which the crystals of 2-[(2S)-4-methyl-2-phenyl-1-piperazinyl]-3-pyridinecarboxylic acid were collected by filtration and dried in a vacuum oven. The yield of the crystals was 20.9 g (57%, ee >99%).
¹H-NMR data (CDCl₃): δ (ppm): 2.43 (s,3H, CH₃), 2.61 (t, 2H, CH₂), 3.12 (m, 3H, CH₂+CH), 3.42 (dt, 1 H, CH), 4.79 (dd, 1 H, CH_{benzyl}), 7.11-7.28 (m, 4H, Ar-H), 8.25 (dd, 1 H, Ar-H), 8.54 (dd, 1 H, Ar-H).
The ee was determined by HPLC analysis: Chiralcel OD-H 250*4.6mmID, 10 µm (Daicel), 10 % iso-propylalcohol in heptane, flow rate 1.0 ml.min⁻¹, UV-detector, column temperature 40 °C.

### A. Preparation of (14bS)-1,3,4,14b-tetrahydro-2-methyl-pyrazino[2,1-a]pyrido[2,3-c][2]benzazepin-10(2H)-one (ketone Compound II)

Thionyl chloride (4.91 ml, 67.3 mmol) was added drop wise to a solution of (10.0 g, 33.6 mmol) 2-(4-methyl-2-phenyl-1-piperazinyl)-3-pyridinecarboxylic acid (compound I) and N,N-dimethylformamide (5 ml) in dichloromethane (100 ml) at about 0 °C under a nitrogen atmosphere. The reaction mixture was stirred at room temperature for one hour. Then aluminum chloride (24.2 g, 181 mmol) was added in portions to the reaction mixture at about 0 °C. The reaction mixture was stirred at room temperature until the reaction was completed.

To the reaction mixture was added water (500 ml), dichloromethane (500 ml) and a solution of sodium hydroxide (36.53 g, 913 mmol) in water (100 ml). The reaction mixture was filtered over dicalite. The organic layer was separated from the water layer. The water layer was extracted twice with dichloromethane (250 ml). The three organic layers were combined and washed twice with water (250 ml). The organic layer was dried with magnesium sulphate, filtered and the solvent was evaporated under vacuum. The brown oil was dissolved in ethanol (170 ml) and 146 ml of the ethanol was evaporated under vacuum. The solid was crystallized from the ethanol for about an hour at room temperature and one hour at about 0 °C. The solid was isolated and dried at 40 °C under vacuum resulting in the title product as a yellow solid (6.54 g, 69.9% yield, defined as mole% relative to the starting compound). Purity according HPLC was 100% (HPLC purity being defined as main peak area divided by total peak area x 100%), the enantiomeric purity according HPLC-chiral was 100% and the melting point according DSC was 150.6 °C. The obtained product is characterised by ¹H-NMR (CDCl₃, 600 MHz) as follows: δ = 2.35 (dt, 1H), 2.44 (s, 3H), 2.55 (dd, 1H), 3.02 (m, 1 H), 3.20 (dt, 1 H), 3.38 (d, 1 H), 4.42 (d, 1 H), 4.90 (m, 1 H), 6.85 (dd, 1 H), 7.35 (dt, 1 H), 7.56 (dt, 1 H), 7.62 (dd, 1 H), 8.02 (d, 1 H), 8.36 (dd, 1 H), 8.51 (dd, 1 H).

### B. Preparation of S-mirtazapine

A solution of aluminum chloride (1.43, 10.7 mmol) and lithium aluminum hydride (32.3 ml, 32.3 mmol) in tetrahydrofuran (21 ml) was added drop wise to a solution of the product of step A (6.00 g, 21.5 mmol) in tetrahydrofuran (18 ml) at about 0 °C under a nitrogen atmosphere. The reaction mixture was stirred at 50 °C for 20 hours.

A solution of sodium tartrate dihydrate (5.96 g, 25.9 mmol) in water (49 ml) was added to the reaction mixture at about 0 °C resulting in a suspension in the water layer. The pH of the solution was set at 14 with sodium hydroxide solution (4 M). The organic layer was separated from the water layer. The water layer was extracted twice with toluene (60 ml). The three organic layers were combined and washed twice with water (60 ml). The organic layer was dried with magnesium sulfate, filtered and the solvent was evaporated under vacuum resulting in the title product in nearly quantitative yield. The product was pure according to NMR. The enantiomeric purity according HPLC-chiral was 100%.

The crude product was dissolved in ethanol (15.8 ml). To this solution a solution of maleic acid (2.75 g, 23.7 mmol) in ethanol (7.9 ml) was added. The solution was stirred during a night at room temperature. The solid was isolated and dried at 40 °C under vacuum and gave a white solid (5.28 g, 70 % yield). Purity according HPLC was 96% and the enantiomeric purity according HPLC-chiral was 100%. **¹H-NMR (CDCl₃, 400 MHz):** δ = 2.98 (s, 3H), 3.30 (dt, 1 H), 3.46 (t, 1 H), 3.53 (d,1 H), 3.55 (m, 1 H), 3.56 (dt, 1 H), 3.68 (m, 1 H), 3.98 (m, 1 H), 4.47 (dd, 1 H), 4.59 (d, 1 H), 6.26 (s, 2H), 6.92 (dd, 1 H), 7.14 (m, 1H), 7.22 (m, 1H), 7.22 (m, 1H), 7.29 (m, 1H), 7.53 (dd, 1H), 8.13 (dd, 1H).

### Example 2

### A.Preparation of (14bS)-1,2,3,4,10,14b- Hexahydro- 2-methylpyrazino [2,1-a]pyrido

### [2,3-c][2]benzazepin-10-ol (hydroxyl compound III)

Lithium aluminum hydride (3.51 ml, 3.51 mmol) was added drop wise to a solution of the product of Example 1A (0.98 g, 3.51 mmol) in tetrahydrofuran (10 ml) at about 0 °C under a nitrogen atmosphere. The reaction mixture was stirred at room temperature for 1 hour.

To the reaction mixture were added water (10 ml), ethyl acetate (10 ml) and a sodium hydroxide solution (8.3 M, 6 ml). The pH of the water layer was 14. The organic layer was separated from the water layer. The water layer was extracted twice with ethyl acetate (10 ml). The three organic layers were combined and washed twice with water (10 ml). The organic layer was dried with magnesium sulfate, filtered and the solvent was evaporated under vacuum resulting in the title product as a light yellow solid (0.987 g, 100%). Purity according HPLC was 95%. Diastereomeric ratio is approximately 85/15. **¹H-NMR (MeOD, 400 MHz):** δ = 2.38 (s, 3H), 2.46 (dt, 1H), 2.62 (dd, 1 H), 2.96-3.03 (2×m, 2H, -1), 3.53 (dt, 1 H), 3.70 (dt, 1 H), 4.42 (d, 1 H), 5.53 (broad s, 1H), 6.89 (dd, 1H), 7.20-7.41 (4H,-7-8-9-10), 7.59 (dd,1H), 8.12 (dd, 1H).

### B. Preparation of S-mirtazapine

Lithium aluminium hydride (3.3 ml, 1M in THF) was added dropwise to a solution of aluminium chloride (143 mg, 1.07 mmol) in tetrahydrofuran (15 ml) at 0°C. After 15 minutes a solution of the compound III obtained in Example 2A in tetrahydrofuran (10 ml) was added. The reaction mixture was heated to 50°C for 18 hours. When the reaction was completed, a solution of sodium tartrate dihydrate (1 g, 4.3 mmol) in water (10 ml) was added to the reaction mixture at about 0 °C resulting in a suspension in the water layer. The pH of the solution was set at 14 with sodium hydroxide solution (4 M). The organic layer was separated from the water layer. The water layer was extracted twice with toluene (10 ml). The three organic layers were combined and washed twice with water (10 ml). The organic layer was dried with magnesium sulfate, filtered and the solvent was evaporated under vacuum resulting in the title product (0.24 g, quantitative). The product was pure according to NMR and HPLC. The enantiomeric purity according HPLC-chiral was 100%.

### Example 3

### A. Preparation of (14bS)-1,3,4,14b-tetrahydro-2-methyl-pyrazino[2,1-a]pyrido[2,3-c][2]benzazepin-10(2H)-one (Compound II)

Thionyl chloride (22 ml, 301 mmol) was added drop wise to a solution of 2-(4-methyl-2-phenyl-1-piperazinyl)-3-pyridinecarboxylic acid (compound I, 44 g, 148 mmol) and N,N-dimethylformamide (22 ml) in dichloromethane (440 ml) at about 0 °C under a nitrogen atmosphere. The reaction mixture was stirred at room temperature for 75 minutes. Then aluminum chloride (39.5 g, 296 mmol) was added to the reaction mixture at about 0 °C. Over the course of 8 hours three additional portions of aluminium chloride (each 19.7 g, 148 mmol) were added. The reaction mixture was stirred at room temperature until the reaction was completed.

To the reaction mixture was added water (2200 ml), dichloromethane (1320 ml) and a solution of sodium hydroxide (147 g, 3.7 mol) in water (440 ml). The reaction mixture was filtered over dicalite. The organic layer was separated from the water layer. The water layer was extracted twice with dichloromethane (500 ml). The three organic layers were combined and washed twice with water (600 ml). The organic layer was dried with magnesium sulphate, filtered and the solvent was evaporated under vacuum. The title compound was obtained as an oil (39.7 g, 96% yield)., the crude product was sufficiently pure for use in the following step. The enantiomeric purity according HPLC-chiral was 100%. As opposed to example 1, in example 3 there is no intermediate crystallisation step to isolate compound II. The crude compound II thus obtained was sufficiently pure according to NMR for direct use in the following step to be converted into S-mirtazapine according to the procedure as described in Example 1 step B.

### Example 4

### A. Preparation of mirtazapine

Racemic 1,2,3,4,10,14b-Hexahydro-2-methylpyrazino[2,1-a]pyrido[2,3-c][2]benzazepin-10-ol (100 mg, 0.36 mmol) is dissolved in sulfuric acid (10 ml). To this solution sodium borohydride (135 mg, 3.6 mmol) was added. The reaction mixture was stirred at ambient temperature. After two hours the reaction was completed. To the reaction mixture was added water (10 ml), ethyl acetate (10 ml) and 33% aqueous sodium hydroxide solution (42 ml). After separation of the layers, the water layer was extracted with ethyl acetate (10 ml). The combined ethyl acetate layers were washed three times with water (10 ml) and were subsequently dried with magnesium sulphate and evaporated under vacuum. This furnished the title compound in a yield of 75 mg (80%). The product was identified by NMR and had an HPLC purity of 96%. This example shows that sodium borohydride in sulfuric acid is a good reduction agent for reducing the hydroxyl compound II to mirtazapine.

### Example 5

### A. Preparation of mirtazapine

Racemic hydroxyl compound III (100 mg, 0.36 mmol) is dissolved in methanesulfonic acid (10 ml). To this solution sodium borohydride (135 mg, 3.6 mmol) was added. The reaction mixture was stirred at ambient temperature until completion. To the reaction mixture was added water (10 ml), ethyl acetate (10 ml) and 33% aqueous sodium hydroxide solution (42 ml). After separation of the layers, the water layer was extracted with ethyl acetate (10 ml). The combined ethyl acetate layers were washed three times with water (10 ml) and were subsequently dried with magnesium sulphate and evaporated under vacuum. This furnished the title compound in a yield of 80 mg (85%). The product was identified by NMR and had an HPLC purity of 99%. This example shows that sodium borohydride in methanesulfonic acid is a good reduction agent for reducing the hydroxyl compound II to mirtazapine.

## Claims

1. A method for the preparation of mirtazapine (formula IV) having greater than 50% enantiomeric excess of the R or S form, said method comprising the steps of
a: providing a carboxylic acid compound according to Formula I having greater than 50% enantiomeric excess of the R or S form,
b: converting the carboxylic acid group of compound I into a ketone group, producing a ketone compound of Formula II,
c: optionally reducing ketone compound II with a metal hydride or a borohydride to form the intermediate hydroxy compound of Formula III,
d: forming the mirtazapine of Formula IV by reduction of the ketone compound II or of the hydroxy compound III using as the reducing agent a mixture of a metal hydride and a metal halogenide or a mixture of a borohydride and a strong acid.

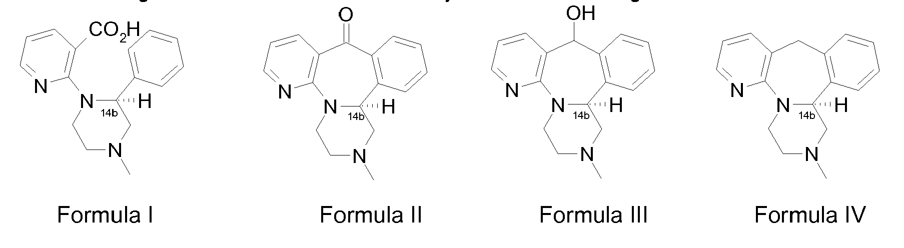

2. The method according to claim 1, wherein the formed mirtazapine has an enantiomeric excess of the R or S form of at least 80% and the obtained mirtazapine product comprises side products in an amount below 10 wt% (wt% expressed as the total weight of side products divided by the total weight of the obtained reaction product x 100%).

3. The method according to claim 2 wherein the enantiomeric excess is at least 95% and the amount of side products is below 2 wt%.

4. The method according to claims 1 to 3, wherein the reduction step (c) is done with a metal hydride as reduction agent.

5. The method according to claims 1 to 3, wherein the reduction in step (d) is done with a mixture of lithium aluminium hydride and an aluminium halogenide as reduction agent.

6. The method according to claims 1-3, wherein the reducing agent in step (d) is mixture of sodium borohydride and methanesulfonic acid or sulphuric acid.

7. The method according to claims 1 to 6, wherein in step (b) the carboxylic acid group is first activated with a chlorinating agent and subsequently reacted with a Lewis acid.

8. The method according to claim 7, wherein the chlorinating agent is thionyl chloride, oxalyl chloride or phosphorus oxychloride.

9. A compound according to formula II in a mixture having an enantiomeric excess of the R or S form of at least 50%, or in enantiopure pure R or S form.

10. A compound according to formula III in a mixture having a substantial an enantiomeric excess of the R or S form of at least 50%, or in enantiopure pure R or S form.

11. The compound according to claim 9 or 10 having enantiomeric excess of the R or S form of at least 80%, and comprising side products in an amount below 10 wt%.

12. The use of any of the compounds according to claims 9 to 11 for the preparation of mirtazapine (formula IV) having greater than 50% enantiomeric excess of the R or S form.

## Patentansprüche

1. Ein Verfahren zur Herstellung von Mirtazapin (Formel IV) mit einem Enantiomerenüberschuss der R- oder S-Form von mehr als 50%, wobei das Verfahren die folgenden Schritte umfasst:
a: Bereitstellen einer Carbonsäureverbindung der Formel I mit einem Enantiomerenüberschuss der R- oder S-Form von mehr als 50%,
b: Umwandeln der Carbonsäuregruppe der Verbindung I in eine Ketongruppe, wobei eine Ketonverbindung der Formel II erzeugt wird,
c: gegebenenfalls Reduzieren der Ketonverbindung II mit einem Metallhydrid oder einem Borhydrid, um die intermediäre Hydroxyverbindung der Formel III zu bilden,
d: Bilden des Mirtazapins der Formel IV durch Reduzieren der Ketonverbindung II oder der Hydroxyverbindung III unter Verwendung einer Mischung aus einem Metallhydrid und einem Metallhalogenid oder einer Mischung aus einem Borhydrid und einer starken Säure als Reduktionsmittel.

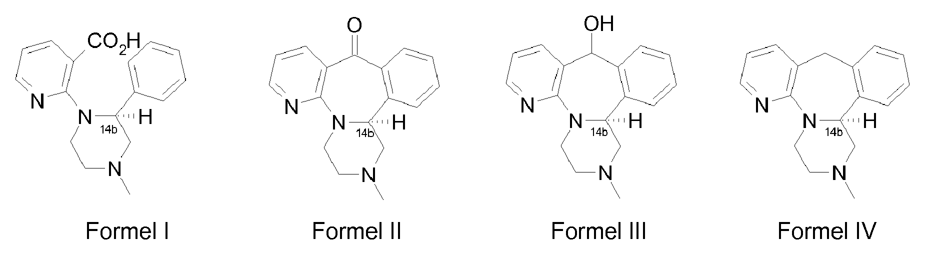

2. Das Verfahren gemäß Anspruch 1, wobei das gebildete Mirtazapin einen Enantiomerenüberschuss der R- oder S-Form von wenigstens 80% besitzt und das erhaltene Mirtazapin-Produkt Nebenprodukte in einer Menge von weniger als 10 Gew.-% umfasst (Gew.-% ausgedrückt als das Gesamtgewicht der Nebenprodukte dividiert durch das Gesamtgewicht des erhaltenen Reaktionsprodukts x 100%).

3. Das Verfahren gemäß Anspruch 2, wobei der Enantiomerenüberschuss wenigstens 95% beträgt und die Menge an Nebenprodukten unter 2 Gew.-% liegt.

4. Das Verfahren gemäß den Ansprüchen 1 bis 3, wobei der Reduktionsschritt (c) mit einem Metallhydrid als Reduktionsmittel durchgeführt wird.

5. Das Verfahren gemäß den Ansprüchen 1 bis 3, wobei die Reduktion in Schritt (d) mit einer Mischung aus Lithiumaluminiumhydrid und einem Aluminiumhalogenid als Reduktionsmittel erfolgt.

6. Das Verfahren gemäß den Ansprüchen 1-3, wobei das Reduktionsmittel in Schritt (d) eine Mischung aus Natriumborhydrid und Methansulfonsäure oder Schwefelsäure ist.

7. Das Verfahren gemäß den Ansprüchen 1 bis 6, wobei in Schritt (b) die Carbonsäuregruppe zunächst mit einem Chlorierungsmittel aktiviert wird und anschließend mit einer Lewissäure umgesetzt wird.

8. Das Verfahren gemäß Anspruch 7, wobei das Chlorierungsmittel Thionylchlorid, Oxalylchlorid oder Phosphoroxychlorid ist.

9. Eine Verbindung gemäß der Formel II in einem Gemisch mit einem Enantiomerenüberschuss der R- oder S-Form von wenigstens 50% oder in enantiomerenreiner R- oder S-Form.

10. Eine Verbindung gemäß der Formel III in einem Gemisch mit wesentlich einem Enantiomerenüberschuss der R- oder S-Form von wenigstens 50% oder in enantiomerenreiner R- oder S-Form.

11. Die Verbindung gemäß Anspruch 9 oder 10, die einen Enantiomerenüberschuss der R- oder S-Form von wenigstens 80% besitzt und Nebenprodukte in einer Menge von weniger als 10 Gew.-% umfasst.

12. Die Verwendung einer der Verbindungen gemäß den Ansprüchen 9 bis 11 zur Herstellung von Mirtazapin (Formel IV) mit einem Enantiomerenüberschuss der R- oder S-Form von mehr als 50%.

## Revendications

1. Procédé pour la préparation de mirtazapine (formule IV) ayant plus de 50% d'excès énantiomère de la forme R ou S, ledit procédé comprenant les étapes consistant
a: à fournir un composé d'acide carboxylique selon la Formule I ayant plus de 50% d'excès énantiomère de la forme R ou S,
b: à convertir le groupe acide carboxylique du composé I en un groupe cétone, produisant un composé de cétone de Formule II,
c: facultativement, à réduire le composé de cétone II avec un hydrure de métal ou un borohydrure pour former le composé hydroxy intermédiaire de Formule III,
d: à former la mirtazapine de Formule IV par réduction du composé de cétone II ou du composé hydroxy III en utilisant en tant qu'agent réducteur un mélange d'un hydrure de métal et d'un halogénure de métal ou un mélange d'un borohydrure et d'un acide fort.

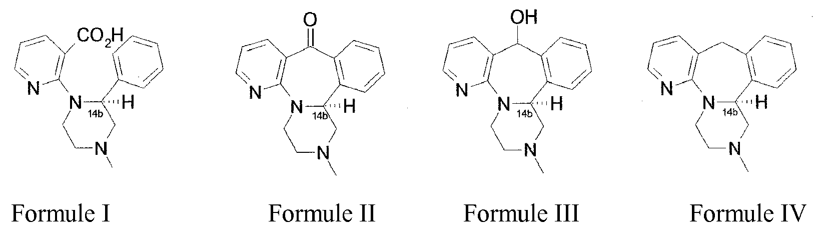

2. Procédé selon la revendication 1, dans lequel la mirtazapine formée a un excès énantiomère de la forme R ou S d'au moins 80% et le produit de mirtazapine obtenu comprend des produits secondaires en une quantité inférieure à 10% en poids (% en poids exprimé comme le poids total des produits secondaires divisé par le poids total du produit de réaction obtenu x 100%).

3. Procédé selon la revendication 2, dans lequel l'excès énantiomère est d'au moins 95% et la quantité de produits secondaires est inférieure à 2% en poids.

4. Procédé selon les revendications 1 à 3, dans lequel l'étape de réduction (c) est effectuée avec un hydrure de métal en tant qu'agent de réduction.

5. Procédé selon les revendications 1 à 3, dans lequel la réduction dans l'étape (d) est effectuée avec un mélange d'hydrure de lithium et d'aluminium et d'un halogénure d'aluminium en tant qu'agent de réduction.

6. Procédé selon les revendications 1 à 3, dans lequel l'agent réducteur dans l'étape (d) est un mélange de borohydrure de sodium et d'acide méthanesulfonique ou d'acide sulfurique.

7. Procédé selon les revendications 1 à 6, dans lequel, dans l'étape (b), le groupe acide carboxylique est d'abord activé avec un agent chlorant puis mis à réagir avec un acide de Lewis.

8. Procédé selon la revendication 7, dans lequel l'agent chlorant est le chlorure de thionyle, le chlorure d'oxalyle ou l'oxychlorure de phosphore.

9. Composé selon la formule II dans un mélange ayant un excès énantiomère de la forme R ou S d'au moins 50%, ou sous une forme R ou S énantiopure.

10. Composé selon la formule III dans un mélange ayant un excès énantiomère substantiel de la forme R ou S d'au moins 50%, ou sous une forme R ou S énantiopure.

11. Composé selon la revendication 9 ou 10, ayant un excès énantiomère de la forme R ou S d'au moins 80%, et comprenant des produits secondaires en une quantité inférieure à 10% en poids.

12. Utilisation de l'un quelconque des composés selon les revendications 9 à 11 pour la préparation d'une mirtazapine (formule IV) ayant plus de 50% d'excès énantiomère de la forme R ou S.
